# EUROPEAN PATENT SPECIFICATION

(11) **EP 4 511 333 B1**
(45) Date of publication and mention of the grant of the patent: **25.03.2026**
(21) Application number: 23725460.2
(22) Date of filing: 21.04.2023
(51) Int. Cl.: C02F 11/04, F17B 1/10, F17B 1/26, F17C 13/02, B09B 3/65, C12M 1/107, C12M 1/00, F17C 1/00, F17B 1/00, C12M 1/34, C12M 3/00

(54) **AN ENERGY OR GAS GENERATION PLANT**
ENERGIE- ODER GASERZEUGUNGSANLAGE
INSTALLATION DE PRODUCTION D'ÉNERGIE OU DE GAZ

(30) Priority: 21.04.2022 NL 2031650
(43) Date of publication of application: 26.02.2025
(73) Proprietor: THE WASTE TRANSFORMERS INVESTMENTS B.V., 2011 CR Haarlem (NL)
(72) Inventor: SUDMEIER, Maarten Floris, 2011 CR Haarlem (NL)
(74) Representative: van Breda, Jacobus
(86) International application number: PCT/NL2023/050214
(87) International publication number: WO 2023/204713

(56) References cited:
- EP-B1- 0 137 833
- CN-U- 202 186 857
- DE-U1- 202006 014 148
- US-A1- 2019 210 902

## Description

The invention relates to an energy or gas generation plant comprising a receiving station for organic waste, optionally a macerator, shredder or cutter connected to the receiving station for processing the organic waste into fragments of organic waste, an aquatic tank for receiving the organic waste or fragments of organic waste, which aquatic tank is connected to an anaerobic digester for digesting the organic waste, wherein a gas holder connects to the anaerobic digester to receive gas from the anaerobic digester, and wherein a power system fuelled by said gas is connected to the gas holder.

Such an energy or gas generation plant, albeit of rather complicated and impractically bulky design, is disclosed by EP-B2 781 589. This known plant has notable disadvantages which are to be avoided, to note a few: the known plant comprises a plurality of small holding tanks in fluid communication with a mixing tank that are configured to perform at least one of a pasteurization and a thermophilic anaerobic digestion on the waste, a large holding tank in fluid communication with the plurality of small holding tanks that is configured to perform mesophilic anaerobic digestion on the waste after at least one of a pasteurization thermophilic anaerobic digestion is performed on the waste. The application of plural small holding tanks followed by a large holding tank makes the design complicated and expensive, not to mention that each of the holding tanks must be accurately controlled to provide the typical process conditions that are required in those tanks, which are moreover different in the small holding tanks on the one part and the large holding tank on the other part.

A further complication is that the known plant comprises a separate large gas storage container comprising a gas storage tank that is configured to store biogas generated by the mesophilic anaerobic digestion in the second large tank. In fact the prior art plant is so bulky that it requires the application of two separate containers to house all the equipment of the plant.

Further relevant disclosures can be found in US 2019/210902 A1, EP 0 137 833 B1, DE 20 2006 014148 U1 or CN 202 186 857 U.

The instant invention therefore has as a first object to reduce the footprint of the energy or gas generation plant, therewith improving its transportability.

As a further objective the invention seeks to overcome the disadvantage of the gas storage tank of the plant requiring for its handling a separate gas storage container.

As a third objective the invention seeks to reduce the bulkiness of the gas storage tank so that it can in principle be placed in one and the same container with the other parts of the energy or gas generation plant, without compromising the storage capacity of the gas storage tank.

It is therefore a further object of the invention to improve the known plant, for which purpose a plant is proposed in accordance with independent claim 1. Preferred embodiments of the invention are defined in the dependent claims.

In a first aspect of a transportable energy or gas generation plant according to the invention, the plant comprises a receiving station for organic waste, an aquatic tank for receiving the organic waste or fragments of organic waste, which aquatic tank is connected to an anaerobic digester for digesting the organic waste, wherein a gas holder connects to the anaerobic digester to receive gas from the anaerobic digester, and wherein a power system fuelled by said gas is connected to the gas holder, wherein the gas holder is embodied with a guiding frame to guide an up-and-down movable weight frame, wherein below the weight frame a flexible bag is provided for receipt and storage of said gas originating from the anaerobic digester, and which flexible bag supports the weight frame, wherein said up-and-down movable weight frame provides a continuous load on the flexible bag so as to enable that a volume content of the flexible bag is changeable whilst maintaining a static pressure in the flexible bag as determined by the up-and-down movable weight frame, and that the gas holder comprises a measurement organ for measuring a height of the weight frame and/or a volume content of the flexible bag, which measurement organ is connected to a controller that drives the power system so as to maintain the height of the weight frame and/or the volume content of the flexible bag at a predetermined value, which is preferably constant. To a large extent this feature avoids the necessity to flare of the generated biogas, which might otherwise go to waste when there is an excessive supply of this biogas. This feature of the invention therefore considerably contributes to the optimization of the environmental circularity of the proposed energy or gas generation plant.

Further the usable capacity of the gas holder as applied in the plant of the invention is therewith tremendously improved. In comparison with prior art solutions space savings are achievable up to 85% of the space that is required to reserve for the gas holder and ancillary equipment according to the prior art. Suitably the guiding frame is embodied with guide rails that cooperate with the up-and-down movable weight frame. This provides for an accurate and smooth up-and-down movability of the weight frame.

Suitably the weight frame is selected to provide the gas in the flexible bag with an overpressure in the range of 30 - 80 mbar. Suitably the measurement organ is a laser based level or distance meter, which provides reliable and accurate measurements.

The invention will hereinafter be further elucidated with reference to the drawing of an exemplary embodiment of a transportable energy or gas generation plant according to the invention that is not limiting as to the appended claims.

In the drawing:
- figure 1 shows a process flow diagram of a plant of the invention;
- figure 2A shows detail features of the gas holder as applied in the plant of the invention in an isometric view and when fully loaded ;
- figure 2b shows detail features of the gas holder as applied in the plant of the invention in a frontal view and when loaded for about 20% of full capacity; and
- figure 3 shows the controller that drives the power system as applied in the plant of the invention.

Whenever in the figures the same reference numerals are applied, these numerals refer to the same parts.

The energy or gas generation plant of the invention and its parts can be dimensioned such that the complete plant is fittable in a standard sea container, so that it can easily be transported.

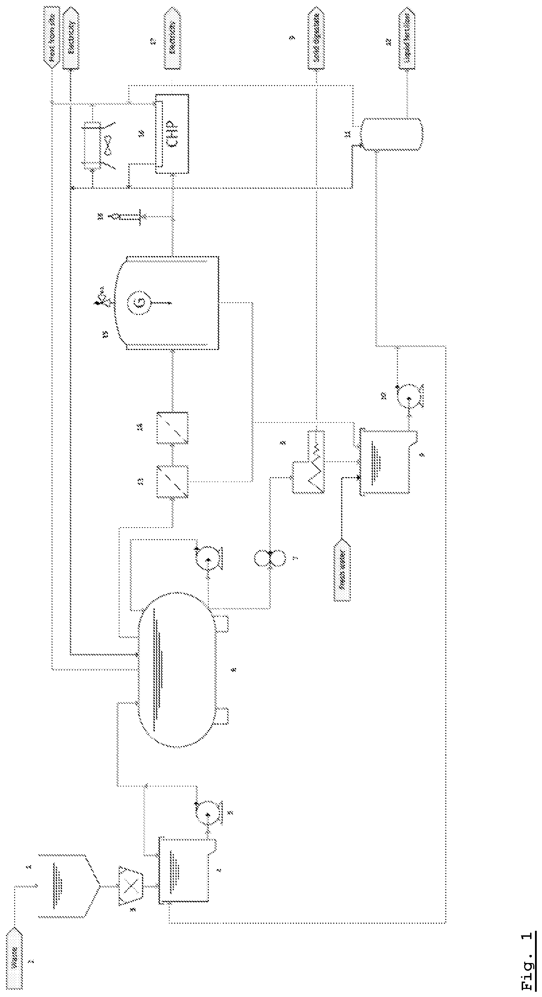

The parts and the functionality of these parts making up the plant of the invention is illustrated in figure 1. Figure 1 shows that there is an entry section in the form of a hopper 1 for instance, which can receive waste 2 for processing the plant. Usually the waste 2 is made to smaller pieces in a macerator, shredder or cutter 3, after which the smaller pieces are transported to a mixing tank 4 filled with water. A pump 5 is used to transport the mixture of water and waste towards an anaerobic digester 6. The anaerobic digester 6 outputs a slurry which is transported with a slurry pump 7 towards a dewatering screw 8 which provides usable solid digestate 9 as one of the outputs of the anaerobic digester 6. A liquid digestate of the slurry leaves the dewatering screw 8 towards a water storage 9. A water pump 10 connects to the water storage 9 in order to transport a major part of the flow towards a pasteurizer 11, which flow after pasteurization eventually comes available as liquid fertilizer 12.

Another output of the anaerobic digester 6 is biogas which after drying in a demister 13 and cleaning in active carbon filter 14, is stored in gas holder 15. Gas from the gas holder 15 fuels a power system 16 which generates electrical energy 17. It is also possible to connect a flare 18 to the gas holder 15 in order to dispense of excess biogas.

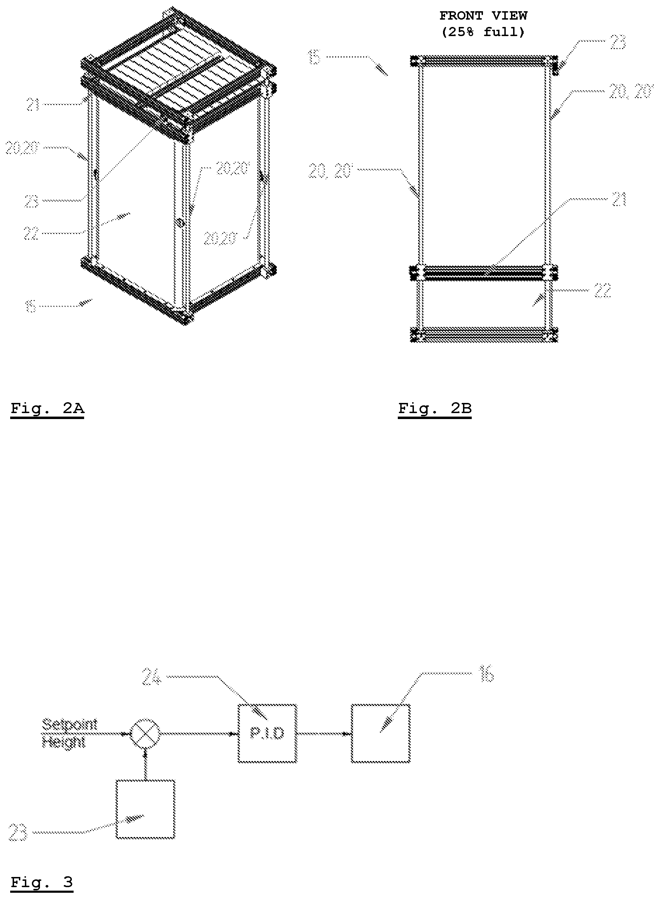

With reference to figures 2A and 2B the construction of the gas holder 15 is further explained. Figure 2A shows the gas holder 15 when full capacity of the gas holder is used. Conversely figure 2B shows the gas holder 15 when approximately 20% of full capacity is used.

As depicted in figures 2A and 2B the gas holder 15 comprises a guiding frame 20 to guide an up-and-down movable weight frame 21, wherein below the weight frame 21 a flexible bag 22 is provided for receipt and storage of gas originating from the anaerobic digester 6. The flexible bag 22 supports the weight frame 21, wherein said up-and-down movable weight frame 21 provides a continuous load on the flexible bag 22 so as to enable that a volume content of the flexible bag 22 is changeable in order to accommodate the supply of gas by the anaerobic digester 6. This is illustrated by comparing figures 2A and 2B, which show that the volume content of the flexible bag 22 is changeable. With different volume content of the flexible bag 22 a static pressure in the flexible bag 22 is maintained as determined by the weight of the up-and-down movable weight frame 21. The weight frame 21 is selected with a weight to provide the gas in the flexible bag 22 with an overpressure in the range of 30-80 mbar.

The guide frame 20 preferably comprises guide rails 20' that cooperate with the up-and-down movable weight frame 21, to enable smooth up-and-down movement of the weight frame.

Figures 2A and 2B further show that the gas holder 15 comprises a measurement organ 23 for measuring a height of the weight frame 21 (which is a measure for a volume content of the flexible bag 22), which measurement organ 23 is connected to a controller 24, such as a PID controller, that drives the power system 16 so as to maintain the height of the weight frame 21 at a predetermined value, which is preferably constant. This is depicted in figure 3. It is preferred that the measurement organ 23 is a laser based level or distance meter. It may also be possible to measure the volume content of the flexible back 22 directly, i.e. without measuring the height of the weight frame 21, but this latter measurement is preferred as it is relatively easy to implement.

Embodiments of the present invention can include every combination of features that are disclosed herein independently from each other. Although the invention has been discussed in the foregoing with reference to an exemplary embodiment of the method of the invention, the invention is not restricted to this particular embodiment which can be varied in many ways without departing from the invention. The discussed exemplary embodiment shall therefore not be used to construe the appended claims strictly in accordance therewith. On the contrary the embodiment is merely intended to explain the wording of the appended claims without intent to limit the claims to this exemplary embodiment. The scope of protection of the invention shall therefore be construed in accordance with the appended claims only, wherein a possible ambiguity in the wording of the claims shall be resolved using this exemplary embodiment.

Unless specifically stated as being "essential" above, none of the various components or the interrelationship thereof are essential to the operation of the invention. Rather, desirable results can be achieved by substituting various components and/or reconfiguration of their relationships with one another.

## Claims

1. A transportable energy or gas generation plant comprising a receiving station (1) for organic waste (2), optionally a macerator, shredder or cutter (3) connected to the receiving station (1) for processing the organic waste (2) into fragments of organic waste, an aquatic tank (4) for receiving the organic waste (2) or fragments of organic waste, which aquatic tank (4) is connected to an anaerobic digester (6) for digesting the organic waste (2), wherein a gas holder (15) connects to the anaerobic digester (6) to receive gas from the anaerobic digester (6), and wherein a power system (16) fuelled by said gas is connected to the gas holder (15), **characterized in that** the gas holder (15) comprises a guiding frame (20) to guide an up-and-down movable weight frame (21), wherein below the weight frame (21) a flexible bag (22) is provided for receipt and storage of said gas originating from the anaerobic digester (6), and which flexible bag (22) supports the weight frame (21), wherein said up-and-down movable weight frame (21) provides a continuous load on the flexible bag (22) so as to enable that a volume content of the flexible bag (22) is changeable whilst maintaining a static pressure in the flexible bag (22) as determined by the up-and-down movable weight frame (21), and that the gas holder (15) comprises a measurement organ (23) for measuring a height of the weight frame (21) and/or a volume content of the flexible bag (22), which measurement organ (23) is connected to a controller (24) that drives the power system (16) so as to maintain the height of the weight frame (21) and/or the volume content of the flexible bag (22) at a predetermined value, which is preferably constant.

2. The transportable plant of claim 1, **characterized in that** the guide frame (20) comprises guide rails (20') that cooperate with the up-and-down movable weight frame (21).

3. The transportable plant of claim 1 or 2, **characterized in that** the weight frame (21) is selected with a weight to provide the gas in the flexible bag (22) with an overpressure in the range of 30-80 mbar.

4. The transportable plant of any one of claims 1-3, **characterized in that** the measurement organ (23) is a laser based level or distance meter.

## Patentansprüche

1. Eine transportable Energie- oder Gaserzeugungsanlage, umfassend eine Empfangsstation (1) für organische Abfälle (2), optional einen mit der Empfangsstation (1) verbundenen Zerkleinerer, Schredder oder Schneider (3) zum Verarbeiten der organischen Abfälle (2) zu Fragmenten organischer Abfälle, einen Wassertank (4) zum Aufnehmen der organischen Abfälle (2) oder Fragmente organischer Abfälle, wobei der Wassertank (4) mit einem anaeroben Fermenter (6) zum Vergären der organischen Abfälle (2) verbunden ist, wobei ein Gasspeicher (15) mit dem anaeroben Fermenter (6) verbunden ist, um Gas aus dem anaeroben Fermenter (6) aufzunehmen, und wobei ein mit dem Gas betriebenes Antriebssystem (16) mit dem Gasspeicher (15) verbunden ist, **dadurch gekennzeichnet, dass** der Gasspeicher (15) einen Führungsrahmen (20) umfasst, um einen auf und ab beweglichen Gewichtsrahmen (21) zu führen, wobei unterhalb des Gewichtsrahmens (21) ein flexibler Beutel (22) zur Aufnahme und Speicherung des aus dem anaeroben Fermenter (6) stammenden Gases bereitgestellt ist, und wobei der flexible Beutel (22) den Gewichtsrahmen (21) stützt, wobei der auf und ab bewegliche Gewichtsrahmen (21) eine kontinuierliche Last auf den flexiblen Beutel (22) ausübt, sodass ein Volumeninhalt des flexiblen Beutels (22) veränderbar ist, während ein statischer Druck in dem flexiblen Beutel (22), der durch den auf- und abwärts beweglichen Gewichtsrahmen (21) bestimmt wird, aufrechterhalten wird, und dass der Gasspeicher (15) ein Messorgan (23) zum Messen einer Höhe des Gewichtsrahmens (21) und/oder eines Volumeninhalts des flexiblen Beutels (22) umfasst, wobei das Messorgan (23) mit einer Steuerung (24) verbunden ist, die das Antriebssystem antreibt (16), um die Höhe des Gewichtsrahmens (21) und/oder das Volumen des flexiblen Beutels (22) auf einem vorbestimmten Wert zu halten, der vorzugsweise konstant ist.

2. Die transportable Anlage nach Anspruch 1, **dadurch gekennzeichnet, dass** der Führungsrahmen (20) Führungsschienen (20') umfasst, die mit dem auf und ab beweglichen Gewichtsrahmen (21) zusammenwirken.

3. Die transportable Anlage von Anspruch 1 oder 2, **dadurch gekennzeichnet, dass** der Gewichtsrahmen (21) mit einem Gewicht ausgewählt ist, um das Gas im flexiblen Beutel (22) mit einem Überdruck im Bereich von 30-80 mbar zu versorgen.

4. Die transportable Anlage nach einem der Ansprüche 1 bis 3, **dadurch gekennzeichnet, dass** das Messorgan (23) ein laserbasierter Höhen- oder Entfernungsmesser ist.

## Revendications

1. Installation transportable de production d'énergie ou de gaz comprenant une station de réception (1) de déchets organiques (2), éventuellement un macérateur, un broyeur ou un coupeur (3) relié à la station de réception (1) pour transformer les déchets organiques (2) en fragments de déchets organiques, une cuve aquatique (4) destinée à recevoir les déchets organiques (2) ou les fragments de déchets organiques, ladite cuve aquatique (4) étant reliée à un digesteur anaérobie (6) pour la digestion des déchets organiques (2), dans laquelle un gazomètre (15) est relié au digesteur anaérobie (6) pour recevoir le gaz provenant du digesteur anaérobie (6), et dans laquelle un système de puissance (16) alimenté par ledit gaz est relié au gazomètre (15), **caractérisée en ce que** le gazomètre (15) comprend un châssis de guidage (20) destiné à guider un châssis de lest mobile verticalement (21), dans laquelle, sous le châssis de lest (21), est disposé un sac flexible (22) destiné à la réception et au stockage dudit gaz provenant du digesteur anaérobie (6), et dans laquelle le sac flexible (22) supporte le châssis de lest (21), ledit châssis de lest mobile verticalement (21) exerçant une charge continue sur le sac flexible (22) de manière à permettre une variation du volume du sac flexible (22) tout en maintenant une pression statique dans le sac flexible (22) déterminée par le châssis de lest mobile verticalement (21), et **en ce que** le gazomètre (15) comprend un organe de mesure (23) destiné à mesurer une hauteur du châssis de lest (21) et/ou un volume du sac flexible (22), ledit organe de mesure (23) étant relié à un contrôleur (24) qui commande le système de puissance (16) de manière à maintenir la hauteur du châssis de lest (21) et/ou le volume du sac flexible (22) à une valeur prédéterminée, de préférence constante.

2. Installation transportable selon la revendication 1, **caractérisée en ce que** le châssis de guidage (20) comprend des rails de guidage (20') coopérant avec le châssis de lest mobile verticalement (21).

3. Installation transportable selon l'une quelconque des revendications 1 ou 2, **caractérisée en ce que** le châssis de lest (21) est choisi avec une masse permettant de conférer au gaz contenu dans le sac flexible (22) une surpression comprise entre 30 et 80 mbar.

4. Installation transportable selon l'une quelconque des revendications 1 à 3, **caractérisée en ce que** l'organe de mesure (23) est un capteur de niveau ou de distance à base de laser.
